# EUROPEAN PATENT APPLICATION

(11) **EP 4 273 876 A1**
(43) Date of publication of application: **08.11.2023**
(21) Application number: 21914569.5
(22) Date of filing: 29.12.2021
(51) Int. Cl.: G16H 40/60

(54) **RADIO FREQUENCY OPERATION LOG PROCESSING METHOD, APPARATUS AND SYSTEM, AND COMPUTER-READABLE STORAGE MEDIUM**

(30) Priority: 31.12.2020 CN 202011642024
(71) Applicant: Hangzhou Broncus Medical Co., Ltd., Hangzhou, Zhejiang 310051 (CN)
(72) Inventor: CUI, Changjie, Hangzhou, Zhejiang 310051 (CN); XU, Hong, Hangzhou, Zhejiang 310051 (CN)
(74) Representative: MERH-IP Matias Erny Reichl Hoffmann Patentanwälte PartG mbB
(86) International application number: PCT/CN2021/142748
(87) International publication number: WO 2022/143837

(57) **Abstract**

A radio frequency operation log processing method, apparatus, and system, and a computer-readable storage medium are provided. The method includes: acquiring, in real time, target running data and radio frequency operation data of a target device running in a radio frequency operation system; generating a running log according to the target running data, and classifying and storing the running log in a running log file according to a preset first classification; generating an operation log according to the radio frequency operation data, and classifying and storing the operation log in an operation log file according to a preset second classification; and acquiring and analyzing the running log file and the operation log file at a preset interval, to obtain maintenance data and operation result data of the radio frequency operation system. By the present invention, the reference of the log file and the systematicity and efficiency in the management of the log file are improved.

## Description

### TECHNICAL FIELD

Embodiments of the present disclosure relates to the technical field of data processing, and particularly to a radio frequency operation log processing method, apparatus, and system, and a computer-readable storage medium.

### DESCRIPTION OF THE PRIOR ART

Radio frequency ablation (RFA) is a common minimally invasive technology for tumors. The principle of radio frequency ablation is to use an alternating high-frequency current with a frequency of less than 30 MHz to cause high-speed oscillations and frictions of ions in tumor tissue, so the radio frequency energy is converted into heat energy, causing coagulative necrosis of tumor cells.

A control device for radio frequency operation, as a computer device, often records some log files. However, the information recorded in these log files is scattered and in disordered management.

### SUMMARY OF THE DISCLOSURE

Embodiments of the present disclosure provide a radio frequency operation log processing method, apparatus, and system, and a computer-readable storage medium, by means of which, the generation, classified management, and analysis of logs of running data and radio frequency operation data of a radio frequency operation system are realized, thereby improving the reference and pertinence of a log file and improving the systematicity and efficiency in the management of the log file.

In an aspect, an embodiment of the present disclosure provides a processing method for a radio frequency operation log, applied in a computer device. The method includes:
acquiring, in real time, target running data and radio frequency operation data of a target device running in a radio frequency operation system;
generating a running log according to the target running data, and classifying and storing the running log in a running log file of the target device according to a preset first classification; and generating an operation log according to the radio frequency operation data, and classifying and storing the operation log in an operation log file of the target device according to a preset second classification; and
acquiring and analyzing the running log file at a preset interval, to obtain maintenance data of the radio frequency operation system; and acquiring and analyzing the operation log file at a preset interval, to obtain operation result data of the radio frequency operation system.

In an aspect, an embodiment of the present disclosure further provides a processing apparatus for a radio frequency operation log. The apparatus includes:
a data acquisition module, configured to acquire, in real time, target running data and radio frequency operation data of a target device running in a radio frequency operation system;
a log classification module, configured to generate a running log according to the target running data, and classify and store the running log in a running log file of the target device according to a preset first classification; and generate an operation log according to the radio frequency operation data, and classify and store the operation log in an operation log file of the target device according to a preset second classification; and
a log analysis module, configured to acquire and analyze the running log file at a preset interval, to obtain maintenance data of the radio frequency operation system; and acquire and analyze the operation log file at a preset interval, to obtain operation result data of the radio frequency operation system.

In an aspect, an embodiment of the present disclosure further provides a computer device. The computer device includes:
a memory and a processor, wherein
the memory stores executable program codes; and
the processor is coupled to the memory, and configured to invoke the executable program codes stored in the memory, and implement the steps of the processing method for a radio frequency operation log according to the above embodiments.

In an aspect, an embodiment of the present disclosure further provides a processing system for a radio frequency operation log. The system includes a server and a plurality of radio frequency operation control devices for respective radio frequency operation systems, wherein
the server is configured to register the plurality of radio frequency operation control devices as nodes in a blockchain network; and
the radio frequency operation control device is configured to implement the processing method for a radio frequency operation log according to the above embodiments.

In an aspect, an embodiment of the present disclosure further provides a non-transitory computer-readable storage medium which stores a computer program. When the computer program is executed by the processor, the processing method for a radio frequency operation log according to the above embodiments is implemented.

In various embodiments according to the present disclosure, a running log and a radio frequency operation log are generated according to target running data and radio frequency operation data of a target device running in a radio frequency operation system acquired in real time; the running log is classified and stored in a running log file of the target device according to a preset first classification, and the operation log is classified and stored in an operation log file of the target device according to a preset second classification; and then the running log file and the operation log file are acquired and analyzed at a preset interval, to obtain maintenance data and operation result data of the radio frequency operation system. By the present invention, the generation, classified management, and analysis of logs of running data and radio frequency operation data of the radio frequency operation system are realized. Because the contents of the log include not only particular running data of the system, but also the radio frequency operation log of the system, the reference and pertinence of the log file is improved. Moreover, by means of classification and periodic analysis, the systematicity and efficiency in the management of the log file are also improved.

### BRIEF DESCRIPTION OF DRAWINGS

In order to describe the technical solutions according to the embodiments of the present disclosure or in the prior art more apparently, the drawings needed to be used in the embodiments or in the prior art will be described briefly below. Apparently, the drawings in the following description show some embodiments of the present disclosure. Other drawings can be obtained by persons of ordinary skill in the art based on these drawings without creative efforts.
FIG. 1 shows an application scenarios of a processing method for a radio frequency operation log according to an embodiment of the present disclosure;
FIG. 2 shows a flow chart of implementing a processing method for a radio frequency operation log according to an embodiment of the present disclosure;
FIG. 3 shows a flow chart of implementing a processing method for a radio frequency operation log according to another embodiment of the present disclosure;
FIG. 4 is a schematic diagram of a processing apparatus for a radio frequency operation log according to an embodiment of the present disclosure;
FIG. 5 is a schematic diagram of a processing apparatus for a radio frequency operation log according to another embodiment of the present disclosure;
FIG. 6 is a schematic diagram showing a hardware structure in a computer device according to an embodiment of the present disclosure; and
FIG. 7 is a schematic diagram of a processing system for a radio frequency operation log according to an embodiment of the present disclosure.

### DDESCRIPTION OF EMBODIMENTS

In order to make the objects, technical solutions and advantages of the embodiments of the present disclosure clearer, the technical solutions according to the embodiments of the present disclosure will be clearly and completely described with reference to drawings in the embodiments of the present disclosure. Apparently, the embodiments described are merely some embodiments, but not all of the embodiments of the present disclosure. All other embodiments obtained by ordinary persons skilled in the art based on the embodiments of the present disclosure without creative efforts shall fall within the protection scope of the present disclosure.

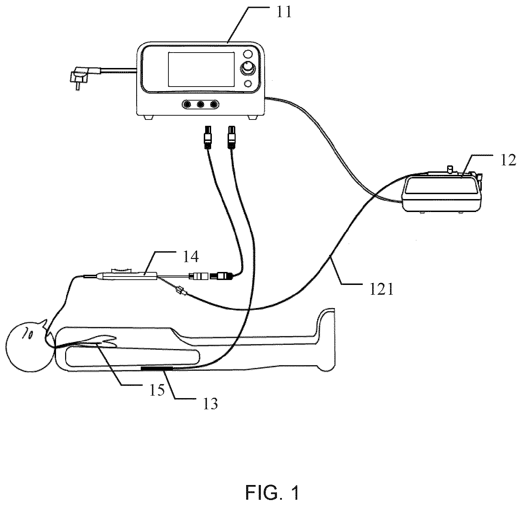

FIG. 1 shows an application scenario of a processing method for a radio frequency operation log according to an embodiment of the present disclosure. As shown in FIG. 1, a radio frequency operation system includes a radio frequency operation control device 11, a syringe pump 12, a neutral electrode 13, a radio frequency ablation catheter 14 and a physical characteristic data acquisition device 15. The physical characteristic data acquisition device includes a temperature acquisition device and an impedance acquisition device.

In an aspect, before the radio frequency operation system starts to run, the physical characteristic data acquisition device 15, an extension tube 121 of the syringe pump 12, and the radio frequency ablation catheter 14 configured to generate and output radio frequency energy are inserted into a radio frequency operation object. Then the neutral electrode 13 is brought into contact with an outer surface of the radio frequency operation object, such that a circuit loop can be formed when a radio frequency current flows through the radio frequency ablation catheter 14, the tissue of the radio frequency operation object and the neutral electrode 13. After a radio frequency operation is triggered, the radio frequency ablation catheter 14 is controlled by the radio frequency operation control device 11 to output a radio frequency current (that is, radio frequency energy) to the radio frequency operation object by discharging, so as to perform the radio frequency operation on the radio frequency operation object. Meanwhile, the syringe pump 12 performs an infusion operation on the radio frequency operation object via the extension tube 121 according to temperature and impedance values acquired by the physical characteristic data acquisition device 15, by infusing physiological saline to the radio frequency operation object, so as to adjust the impedance and the temperature of the radio frequency operation object.

In another aspect, the radio frequency operation control device 11 acquires, in real time, target running data and radio frequency operation data of a target device running in the radio frequency operation system. Then, a running log is generated according to the target running data, and the running log is classified and stored in a running log file of the target device according to a preset first classification; and an operation log is generated according to the radio frequency operation data, and the operation log is classified and stored in an operation log file of the target device according to a preset second classification. Then, the radio frequency operation control device 11 acquires and analyzes the running log file at a preset interval, to obtain maintenance data of the radio frequency operation system; and acquires and analyzes the operation log file at a preset interval, to obtain operation result data of the radio frequency operation system.

It can be understood that the log generation, storage and analysis can also be implemented by a third-party computer device other than the radio frequency operation system, for example, a personal computer or server. After the radio frequency operation system starts to run, various devices therein can establish data connection to the third-party computer device via a wireless network or by wired connection, so as to report the target running data and radio frequency operation data to the third-party computer device.

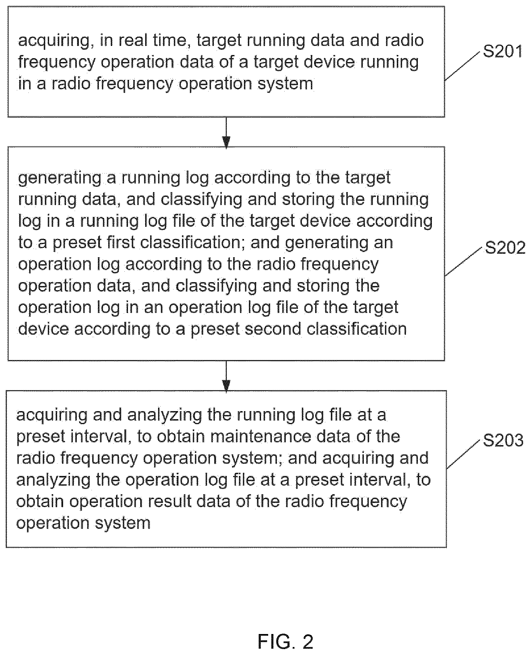

FIG. 2 shows a flow chart of implementing a processing method for a radio frequency operation log according to an embodiment of the present disclosure. The method can be implemented by a computer device, for example, the radio frequency operation control device 11 as shown in FIG. 1, or by a server, a personal computer or other computer device that has established data connection to the target device in a radio frequency operation system. As shown in FIG. 2, the method specifically includes the following steps:

Step S201: acquiring, in real time, target running data and radio frequency operation data of a target device running in a radio frequency operation system.

Specifically, the target running data includes relevant data generated when the target device is in a preset state during the running process. The preset state can include, but is not limited to, for example, a preset target state during normal running, an abnormal state and an error state.

The radio frequency operation data includes real-time radio frequency operation data and periodic radio frequency operation data.

The real-time radio frequency operation data includes operation parameter data invoked in real time by the target device (for example, real-time power of the radio frequency ablation catheter, real-time flow rate of the syringe pump, among others) and physical characteristic data of the radio frequency operation object acquired in real time by the physical characteristic data acquisition device throughout the entire radio frequency operation process.

The periodic radio frequency operation data includes operation parameter data invoked by the target device in preset multiple target stages of the radio frequency operation, and physical characteristic data of the radio frequency operation object acquired in real time by the physical characteristic data acquisition device in the multiple target stages.

The preset multiple target stages of the radio frequency operation can include, but is not limited to, an implementation starting stage, an implementation suspending stage, and an implementation ending stage. The physical characteristic data acquisition device includes a temperature acquisition device and an impedance acquisition device.

The target device is at least one device in the radio frequency operation system.

When the implementing body in this embodiment is an additional computer device outside the radio frequency operation system, the target device in the radio frequency operation system establishes data connection to the additional computer device, and then transmits its own target running data and radio frequency operation data in real time to the additional computer device.

When the implementing body in this embodiment is a device in the radio frequency operation system, the target device may include this device. For example, when the radio frequency operation control device is used as the implementing body, the radio frequency operation control device itself may serve as the target device, or at least one other device (for example, an syringe pump, a radio frequency ablation catheter, among others) in the radio frequency operation system than the radio frequency operation control device can be used as the target device, or the radio frequency operation control device and the at least one other device in the radio frequency operation system can be used as the target devices.

Optionally, when the target device includes the at least one other device, the at least one other device establishes data connection to the radio frequency operation control device, and then transmits its own target running data and radio frequency operation data in real time to the radio frequency operation control device.

The target device can detect whether it is in the preset state, and whether it is in the target stage by using an event detector. For example, when an event that the target device is in the preset state, or a preset event for triggering the starting, suspending, or ending of the implementation of a radio frequency operation (for example, an event that a user presses a physical button for triggering the starting, suspending, or ending of the implementation of the radio frequency operation, an even that the system time reaches a preset radio frequency operation ending time, and so on) is detected by the event detector, the target device is triggered to acquire its own target running data and radio frequency operation data, and record them as a log.

Step S202: generating a running log according to the target running data, and classifying and storing the running log in a running log file of the target device according to a preset first classification; and generating an operation log according to the radio frequency operation data, and classifying and storing the operation log in an operation log file of the target device according to a preset second classification.

Particularly, classification key words for the first classification and the second classification are preset in the computer device. The running log is generated according to the target running data, the type of the running log is determined according to the classification key word for the first classification, and then the running log is stored in the preset running log file of the target device according to the determined type.

Similarly, the operation log is generated according to the radio frequency operation data, the type of the operation log is determined according to the classification key word for the second classification, and then the operation log is stored in the preset operation log file of the target device according to the determined type.

The running log file and the operation log file can be locally stored in the computer device, or to a cloud network.

Step S203: acquiring and analyzing the running log file at a preset interval, to obtain maintenance data of the radio frequency operation system; and acquiring and analyzing the operation log file at a preset interval, to obtain operation result data of the radio frequency operation system.

Particularly, the maintenance data is used to provide a reference for the maintenance and service of software and hardware of the devices in the radio frequency operation system. The operation result data is used to provide a reference for the function and effect of radio frequency operation parameter configurations of the devices in the radio frequency operation system and for the case analysis.

In the embodiment of the present disclosure, a running log and a radio frequency operation log are generated according to target running data and radio frequency operation data of a target device running in a radio frequency operation system acquired in real time; the running log is classified and stored in a running log file of the target device according to a preset first classification; the operation log is classified and stored in an operation log file of the target device according to a preset second classification; and then the running log file and the operation log file are acquired and analyzed at a preset interval, to obtain maintenance data and operation result data of the radio frequency operation system. By the present embodiment, the generation, classified management, and analysis of logs of running data and radio frequency operation data of the radio frequency operation system are realized. Because the contents of the log include not only particular running data of the system, but also the radio frequency operation log of the system, the reference and pertinence of the log file is improved. Moreover, by means of classification and periodic analysis, the systematicity and efficiency in the management of the log file are also improved.

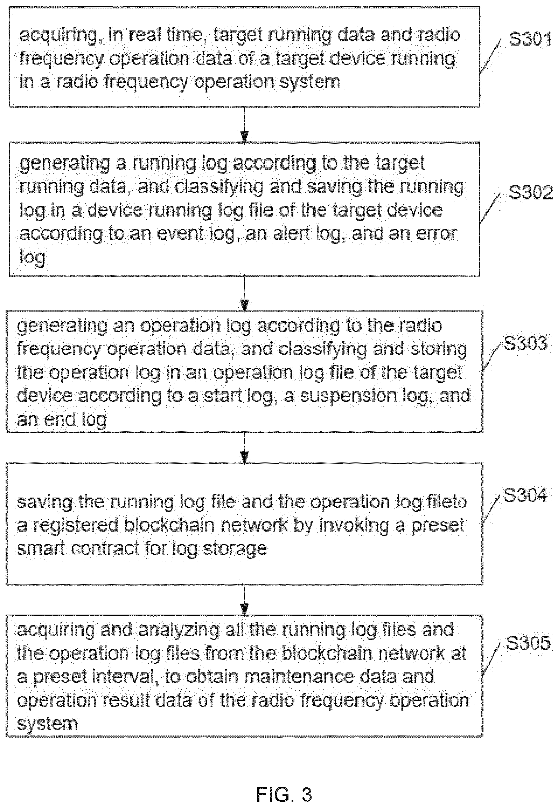

FIG. 3 shows a flow chart of implementing a processing method for a radio frequency operation log according to an embodiment of the present disclosure. The method can be implemented by a computer device, for example, the radio frequency operation control device 11 as shown in FIG. 1, or by a server, a personal computer or other computer device that has established data connection to the target device in a radio frequency operation system. As shown in FIG. 3, the method specifically includes the following steps:

Step S301: acquiring, in real time, target running data and radio frequency operation data of a target device running in a radio frequency operation system.

In this embodiment, Step S301 is similar to Step S201 in the embodiment shown in FIG. 2, and details can refer to the relevant description in the embodiment shown in FIG. 2, and will not be repeated here again.

Step S302: generating a running log according to the target running data, and classifying and storing the running log in a device running log file of the target device according to an event log, an alert log, and an error log.

Specifically, the running log is generated according to the target running data, and the running log is classified and stored in the device running log file of the target device according to a preset first classification. The first classification includes the event log, the alert log and the error log.

The event log includes running data of an event with a preset target level triggered during normal running of the target device. The event with the preset target level may include, but is not limited to, startup, shutdown, modification of device parameters, change of device state, and other events marked with an important level.

Optionally, different levels are preset for various events that may be triggered in the operating system of the computer device, for example: not important, less important, important, and very important, or low, moderate, high, and special. When an event is detected, by the event detector, to be triggered in the system, the level of the triggered event can be acquired by querying the preset level configuration file.

The alert log includes running data when the device is in a preset abnormal state, including the time when the alert is issued, the triggering factor, and whether a target error of the radio frequency operation system is resulted. The preset abnormal state may include, but is not limited to, inputting invalid parameters, abnormal state, and performing invalid operations.

The error log includes data when various errors occur in the device, including the time when the error occurs, the component where the error occurs, the operation stage when the error occurs, the error name, among others. The error will cause the device to stop operation, for example, a peripheral error which cannot be fixed.

Step S303: generating an operation log according to the radio frequency operation data, and classifying and storing the operation log in an operation log file of the target device according to a start log, a suspension log, and an end log.

Specifically, the operation log is generated according to the radio frequency operation data, and the operation log is classified and stored in the operation log file of the target device according to a preset second classification. The second classification includes the start log, the suspension log, and the end log.

The start log includes operation parameter data of the target device when a radio frequency operation is started, and first physical characteristic data of a radio frequency operation object acquired by a temperature and/or impedance acquisition device, for example, a power parameter of a radio frequency ablation catheter when a radio frequency operation is started, an initial flow rate of an syringe pump when the radio frequency operation is started, an initial impedance value, acquired by an impedance acquisition device, of the radio frequency operation object when the radio frequency operation is started, and an initial temperature value, acquired by a temperature acquisition device, of the radio frequency operation object when the radio frequency operation is started.

The suspension log includes operation parameter data of the target device when the radio frequency operation is suspended, and second physical characteristic data of a radio frequency operation object acquired by the temperature and/or impedance acquisition device. For example, a power parameter of a radio frequency ablation catheter when a radio frequency operation is suspended, a present flow rate of an syringe pump when the radio frequency operation is suspended, a present impedance value, acquired by an impedance acquisition device, of the radio frequency operation object when the radio frequency operation is suspended, and a present temperature value, acquired by a temperature acquisition device, of the radio frequency operation object when the radio frequency operation is suspended.

The end log includes operation parameter data of the target device when the radio frequency operation is ended, and third physical characteristic data of a radio frequency operation object acquired by the temperature and/or impedance acquisition device. For example, a power parameter of a radio frequency ablation catheter when a radio frequency operation is ended, a present flow rate of an syringe pump when the radio frequency operation is ended, a present impedance value, acquired by an impedance acquisition device, of the radio frequency operation object when the radio frequency operation is ended, and a present temperature value, acquired by a temperature acquisition device, of the radio frequency operation object when the radio frequency operation is ended.

It can be understood that due to different functions of various devices, the radio frequency operation specifically includes different operations. For example, for the radio frequency ablation catheter, the radio frequency operation includes outputting radio frequency energy to the radio frequency operation object. For the syringe pump, the radio frequency operation includes infusing liquid to the radio frequency operation object. For the radio frequency operation control device, the radio frequency operation is different depending on different controlled objects. When the controlled object is only the radio frequency ablation catheter, the radio frequency operation of the radio frequency operation control device only includes controlling the radio frequency ablation catheter to output radio frequency energy. When the controlled object further includes the syringe pump, the radio frequency operation of the radio frequency operation control device further includes infusing liquid to the radio frequency operation object.

Optionally, the running log file and the operation log file are saved in a file allocation table (FAT) system. The FAT system provides a convenient log access interface, to facilitate the access of the log files. The FAT system can be configured locally in the computer device.

Optionally, the storage structure of the running log file and the operation log file includes a timestamp field, a type field and a content field. The timestamp field is configured to store a timepoint corresponding to the log, the type field is configured to store classification information of the log, and the content field is configured to store the target running data or the radio frequency operation data.

The timepoint corresponding to the log is a timepoint when a present log is recorded. The unit can be set to second or millisecond as needed. Optionally, the timepoint is a time difference from a preset timepoint. The preset timepoint is, for example, the startup time, the time when the radio frequency operation is performed, and so on, as the user specifically defined. The time difference from the preset timepoint is a relative time. Compared with the case where the system time, as an absolute time, is used as a timepoint for recording the present log, the relative time is more accurate when used as a timepoint for recording the present log.

Further, the storage structure of the running log file and the operation log file further includes a description field. The description field is configured to store descriptive information of a present log, and may include, but is not limited to, at least one piece of information selected from the cause to generate the log, the time of triggering the log generation, ID information of the device involved in the log, the running environment of the target device when the log is generated, and the task being performed, etc. For example, at 12:25 on December 25, 2020, a log is generated due to syringe pump failure, and a countdown task is being performed when the failure occurs.

It should be noted that the sequence of performing Step S302 and Step S303 is not limited by the sequence of numbering of the steps. That is, Step S302 and Step S303 may be performed in multiple threads at the same time, or Step S302 may be performed before Step S303, or Step S303 may be performed before Step S302.

Step S304: saving the running log file and the operation log file to a registered blockchain network by invoking a preset smart contract for log storage.

In this embodiment, the computer device is a node in the blockchain network. The smart contract for log storage is a computer protocol consisting of automation script codes, and also an event-driven computer program that can automatically implement a log file storage operation according to a preset contract trigger condition by using a state machine.

The content of the smart contract for log storage is log storage data. The contract trigger condition may include, but is not limited to, a first trigger condition for triggering the operation of sending the running log and the operation log to the blockchain network, a second trigger condition for triggering the operation of storing the running log file and the operation log file by a log file storage side, and a third trigger condition for triggering the operation of providing the log file query.

The first trigger condition is met when new running log file and operation log file are generated, or the running log file and the operation log file are updated or sent at a preset interval. Therefore, by using the first trigger condition, the running log file and the operation log file are automatically saved to the blockchain network when the preset condition is met, thus improving the timeliness of data storage, without giving an opportunity to the tamper, and further improving the security of data.

The second trigger condition may include, but is not limited to, identity condition, device performance condition and motivation condition. The identity condition includes for example, a registered user of the blockchain network reaching a preset age, and use of the device (for example, whether it is used for a single purpose, or whether it is the same kind of device as that uploads the running log file and the operation log file) etc. The device performance condition includes, for example, storage space reached, processor performance reached, and so on. Therefore, by using the second trigger condition, better storage resources can be obtained, so the efficiency of data storage and query is further improved.

The third trigger condition may include, but is not limited to, identity condition of a querier, for example, a first node in the blockchain network, or a computer device able to return designated response information. Because the operation log file generally involves personal privacy of the radio frequency operation object, the security of personal privacy can be ensured by using the third trigger condition.

Particularly, before the running log file and the operation log file are saved to the blockchain network, the computer device is required to be registered as a user of the blockchain network by a register server in the blockchain network, so as to become a node in the blockchain network. Then, the computer device uses a private key returned by the register server to sign the smart contract for log storage, and sends the signed smart contract for log storage to the blockchain network. It can be understood that the smart contract for log storage requires the signature of all node devices in the blockchain network.

Preferably, to prevent the local smart contract for log storage from being tampered, and further ensure the security of data storage, the computer device needs to obtain the smart contract for log storage from the blockchain network each time before the running log and the operation log are uploaded to the blockchain network.

Particularly, the computer device automatically runs the smart contract for log storage after being started, so as to upload and save the running log file and the operation log file to the registered blockchain network by invoking the smart contract for log storage, when the first trigger condition agreed in the smart contract for log storage is met.

Further, when a log file storage request sent from other nodes in the blockchain network is received, the computer device stores the running log file and the operation log file indicated by the log file storage request locally, according to the smart contract for log storage. Particularly, by invoking the smart contract for log storage, the computer device stores the running log file and the operation log file indicated by the log file storage request locally when the second trigger condition agreed in the smart contract for log storage is met.

Further, when a log file query request sent from other nodes in the blockchain network is received, the computer device returns response information including the running log file and the operation log file indicated by the log file query request according to the smart contract for log storage. Particularly, when a log file query request sent from other nodes in the blockchain network is received, the computer device returns response information including the running log file and the operation log file indicated by the log file query request, by invoking the smart contract for log storage, when the third trigger condition agreed in the smart contract for log storage is met.

Step S305: acquiring and analyzing all the running log files and the operation log files from the blockchain network at a preset interval, to obtain maintenance data and operation result data of the radio frequency operation system.

Particularly, the computer device sends a log file query request to all nodes in the blockchain network at a preset interval, and acquires and analyzes all the running log files and the operation log files saved in the blockchain network, to obtain maintenance data and operation result data of the radio frequency operation system.

The maintenance data is used to provide a reference for the maintenance and service of software and hardware of the devices in the radio frequency operation system. The operation result data is used to provide a reference for the function and effect of radio frequency operation parameter configurations of the devices in the radio frequency operation system and for the case analysis.

It can be understood that when the node in the blockchain network includes only the computer device as the device in the radio frequency operation system, the acquired all running log files and operation log files include all running log files and operation log files of the target device in the radio frequency operation system to which the computer device belongs. When the nodes of the blockchain network include devices in multiple different radio frequency operation systems, the acquired all running log files and operation log files include all running log files and operation log files of the target devices in the multiple different radio frequency operation systems.

Further, when the running log file and the operation log file are saved to the blockchain network to which the computer device is registered, the descriptive information of the target device corresponding to the running log file and the operation log file is saved to the blockchain network at the same time. The descriptive information of the target device may include, but is not limited to, the model, hardware configuration among others of the target device. Therefore, different types of target devices can be analyzed according to the descriptive information during analysis.

Particularly, analyzing all the running log files and the operation log files includes, for example, analyzing the alert log and the error log in the running log file. Analyzing the alert log is to analyze the data that causes a target error. Through the above analysis, the name of error occurring in the radio frequency operation system, the time when the error occurs, the component where the error occurs, and so on are obtained. The same errors are classified and statistically counted. If in a preset statistical time period, one or more identical errors occur at the same component for a preset number of times, it is determined that the component needs to be repaired, and maintenance data of the radio frequency operation system is accordingly outputted. The maintenance data includes the component that needs to be repaired, the device corresponding to the component, the present location information of the device and the present operator of the device.

The start log, the suspension log, and the end log in the operation log file are analyzed. Through the above analysis, if information that the radio frequency operation system does not suspend or end the radio frequency operation in advance is obtained, it is determined that the radio frequency operation result is normal, with a desired effect. If information that the radio frequency operation system suspends or ends the radio frequency operation in advance is obtained, it is determined that the radio frequency operation result is abnormal, with unsatisfied effect. Whether the radio frequency operation is ended in advance can be determined by comparing with a preset time required for the normal completion of the radio frequency operation process.

In the embodiment of the present disclosure, a running log and a radio frequency operation log are generated according to target running data and radio frequency operation data of a target device running in a radio frequency operation system acquired in real time; the running log is classified and stored in a running log file of the target device according to a preset first classification, and the operation log is classified and stored in an operation log file of the target device according to a preset second classification; and then the running log file and the operation log file are acquired and analyzed at a preset interval, to obtain maintenance data and operation result data of the radio frequency operation system. By the present embodiment, the generation, classified management, and analysis of logs of running data and radio frequency operation data of the radio frequency operation system are realized. Because the contents of the log include not only particular running data of the system, but also the radio frequency operation log of the system, the reference and pertinence of the log file is improved. Moreover, by means of classification and periodic analysis, the systematicity and efficiency in the management of the log file are also improved. Further, by saving the log files to the blockchain network, the security of the log file storage is improved, to avoid the malicious tampering.

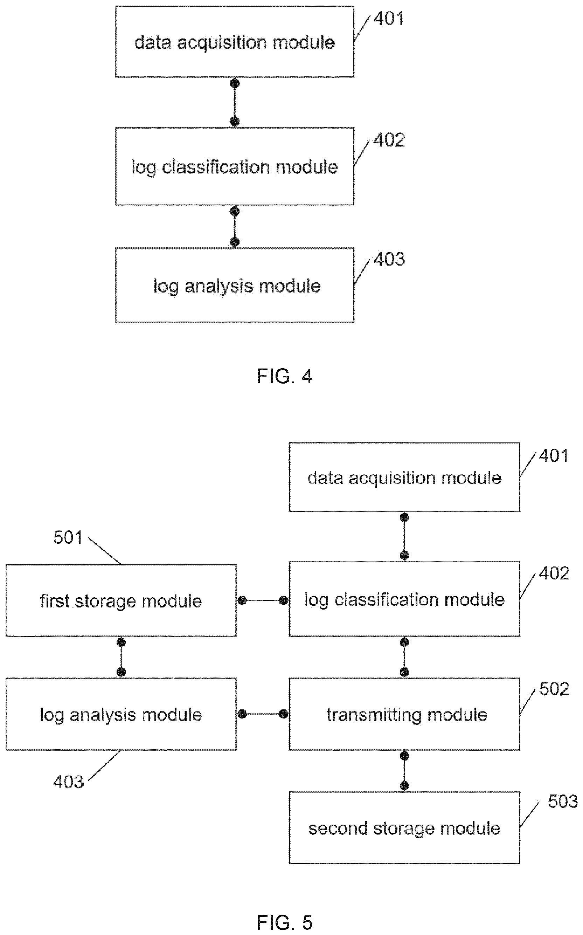

FIG. 4 is a schematic diagram of a processing apparatus for a radio frequency operation log according to an embodiment of the present disclosure. For illustration, only the parts relevant to the embodiments of the present disclosure are shown. The apparatus may be a computer device, or a software module configured in the computer device. As shown in FIG. 4, the apparatus includes a data acquisition module 401, a log classification module 402 and a log analysis module 403.

The data acquisition module 401 is configured to acquire, in real time, target running data and radio frequency operation data of a target device running in a radio frequency operation system.

The log classification module 402 is configured to generate a running log according to the target running data, and classify and store the running log in a running log file of the target device according to a preset first classification, and generate an operation log according to the radio frequency operation data, and classify and store the operation log in an operation log file of the target device according to a preset second classification.

The log analysis module 403 is configured to acquire and analyze the running log file at a preset interval, to obtain maintenance data of the radio frequency operation system; and acquire and analyze the operation log file at a preset interval, to obtain operation result data of the radio frequency operation system.

The specific process for the above modules to achieve their respective functions can refer to the relevant description in the embodiments shown in FIG. 2 and FIG. 3, and will not be repeated here again.

In the embodiment of the present disclosure, a running log and a radio frequency operation log are generated according to target running data and radio frequency operation data of a target device running in a radio frequency operation system acquired in real time; the running log is classified and stored in a running log file of the target device according to a preset first classification, and the operation log is classified and stored in an operation log file of the target device according to a preset second classification; and then the running log file and the operation log file are acquired and analyzed at a preset interval, to obtain maintenance data and operation result data of the radio frequency operation system. By the present embodiment, the generation, classified management, and analysis of logs of running data and radio frequency operation data of the radio frequency operation system are realized. Because the contents of the log include not only particular running data of the system, but also the radio frequency operation log of the system, the reference and pertinence of the log file is improved. Moreover, by means of classification and periodic analysis, the systematicity and efficiency in the management of the log file are also improved.

FIG. 5 is a schematic diagram of a processing apparatus for a radio frequency operation log according to another embodiment of the present disclosure. For illustration, only the parts relevant to the embodiments of the present disclosure are shown. The embodiment as shown in FIG. 5 differs from the embodiment shown in FIG. 4 in:

Optionally, the first classification includes an event log, an alert log, and an error log. The log classification module 402 is specifically configured to classify and save the running log in the running log file of the target device according to the event log, the alert log, and the error log.

The event log includes running data of an event with a preset target level triggered during normal running of the target device.

The alert log includes running data when the target device is in a preset abnormal state.

The error log includes data when an error occurs in the target device.

Optionally, the second classification includes a start log, a suspension log, and an end log. The log classification module 402 is further specifically configured to classify and save the operation log in the operation log file of the target device according to the start log, the suspension log, and the end log.

The start log includes operation parameter data of the target device when a radio frequency operation is started, and first physical characteristic data of a radio frequency operation object acquired by a temperature and/or impedance acquisition device.

The suspension log includes operation parameter data of the target device when the radio frequency operation is suspended, and second physical characteristic data of the radio frequency operation object acquired by the temperature and/or impedance acquisition device.

The end log includes operation parameter data of the target device when the radio frequency operation is ended, and third physical characteristic data of the radio frequency operation object acquired by the temperature and/or impedance acquisition device.

Optionally, the running log file and the operation log file include a timestamp field, a type field and a content field.

The timestamp field is configured to store a timepoint corresponding to the log, the type field is configured to store classification information of the log, and the content field is configured to store the target running data or the radio frequency operation data.

Optionally, the timepoint is a time difference from a preset timepoint.

Optionally, the device further includes:
a first storage module 501, configured to store the running log file and the operation log file in a file allocation table system.

Optionally, the apparatus is an apparatus in the radio frequency operation system, and the apparatus is a node in the blockchain network. The apparatus further includes:
a transmitting module 502 configured to save the running log file and the operation log file to a blockchain network to which the apparatus is registered by invoking a preset smart contract for log storage; and
a log analysis module 403 specifically configured to acquire and analyze all the running log files and the operation log files from the blockchain network at a preset interval, to obtain maintenance data and operation result data.

Optionally, the apparatus further includes:
a second storage module 503, configured to, when a log file storage request sent from other nodes in the blockchain network is received, store the running log file and the operation log file indicated by the log file storage request locally according to the smart contract for log storage.

The transmitting module 502 is further configured to, when a log file query request sent from other nodes in the blockchain network is received, return response information including the running log file and the operation log file indicated by the log file query request according to the smart contract for log storage.

The specific process for the above modules to achieve the respective functions can refer to the relevant description in the embodiments shown in FIG. 2 and FIG. 3, and will not be repeated here again.

In the embodiment of the present disclosure, a running log and a radio frequency operation log are generated according to target running data and radio frequency operation data of a target device running in a radio frequency operation system acquired in real time; the running log is classified and stored in a running log file of the target device according to a preset first classification, and the operation log is classified and stored in an operation log file of the target device according to a preset second classification; and then the running log file and the operation log file are acquired and analyzed at a preset interval, to obtain maintenance data and operation result data of the radio frequency operation system. By the present embodiment, the generation, classified management, and analysis of logs of running data and radio frequency operation data of the radio frequency operation system are realized. Because the contents of the log include not only particular running data of the system, but also the radio frequency operation log of the system, the reference and pertinence of the log file is improved. Moreover, by means of classification and periodic analysis, the systematicity and efficiency in the management of the log file are also improved. Further, by saving the log files to the blockchain network, the security of the log file storage is improved, to avoid the malicious tampering.

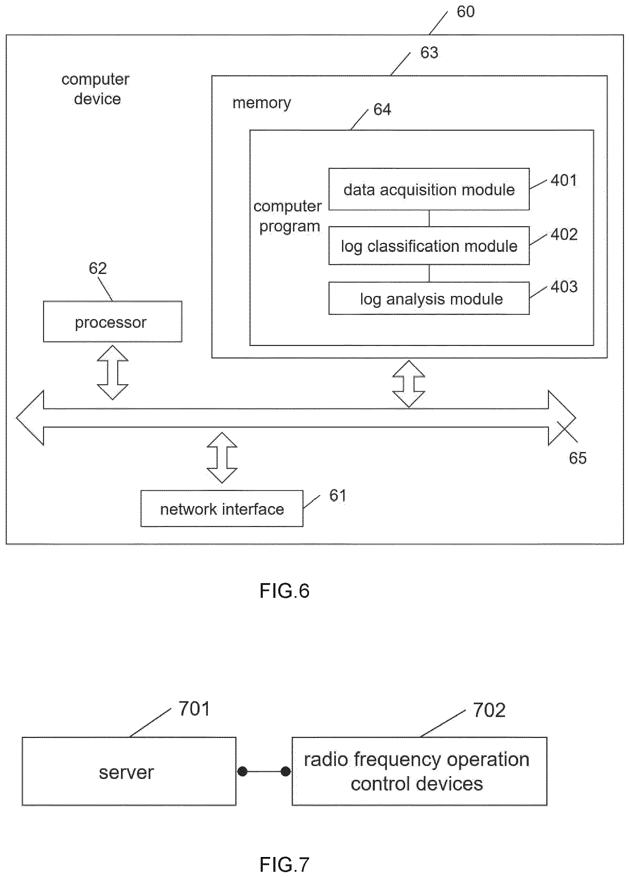

FIG. 6 is a schematic diagram showing a hardware structure in a computer device according to an embodiment of the present disclosure. As shown in FIG. 6, the computer device 60 includes a network interface 61, a processor 62, a memory 63, a computer program 64 stored in the memory 63 and running in the processor 62, and a system bus 65. The system bus 65 is connected to the network interface 61, the processor 62 and the memory 63. When the computer program 64 is executed by the processor 62, steps of the processing method for a radio frequency operation log in various embodiments, for example, Step S201 to Step S203 shown in FIG. 2, are implemented.

The network interface 61 is configured to communicate with other servers.

Exemplarily, the processor 62 may be a central processing unit (CPU) or other general-purpose processor, a digital signal processor (DSP), an application specific integrated circuit (ASIC), a field-programmable gate array (FPGA) or other programmable logic device, a discrete gate or transistor logic device, a discrete hardware assembly, among others. A general-purpose processor can be a microprocessor or any conventional processor.

Exemplarily, the memory 63 is, for example, a hard drive memory, a non-transitory memory, a non-volatile memory (such as flash memory or other electronically programmable limited-erasable memory for forming solid-state drive, etc.), and a volatile memory (such as static or dynamic random access memory), which is not limited in the embodiments of the present disclosure. The memory 63 can include both an internal storage unit in the computer device 60 and an external storage device. The memory 63 is configured to store the computer program and other programs and data required by the computer device 60. The memory 63 may also be configured to temporarily store data that have been outputted or will be outputted.

Exemplarily, the computer program 64 can be divided into one or more modules/units, stored in the memory 63 and executed by the processor 62, to accomplish the present invention. The one or more modules/units can be a series of computer program instruction segments capable of implementing particular functions, and configured to describe an execution process of the computer program 64 in the computer device 60. For example, the computer program 64 can be divided into a data acquisition module 401, a log classification module 402 and a log analysis module 403. The functions of various modules are as described below.

The data acquisition module 401 is configured to acquire, in real time, target running data and radio frequency operation data of a target device running in a radio frequency operation system.

The log classification module 402 is configured to generate a running log according to the target running data, and classify and store the running log in a running log file of the target device according to a preset first classification, and generate an operation log according to the radio frequency operation data, and classify and store the operation log in an operation log file of the target device according to a preset second classification.

The log analysis module 403 is configured to acquire and analyze the running log file at a preset interval, to obtain maintenance data of the radio frequency operation system; and acquire and analyze the operation log file at a preset interval, to obtain operation result data of the radio frequency operation system.

Optionally, the computer program 64 further includes, for example, a first storage module 501, a transmitting module 502 and a second storage module 503.

The specific process for the above functional modules to achieve the respective functions can refer to relevant descriptions in embodiments shown in FIGS. 4 to 5, and will not be repeated here again.

Further, the memory 63 further stores a device driver, which may be a network and interface driver.

It can be understood by those skilled in the art that FIG. 6 is merely an example of the computer device 60, and does not constitute a limitation on the computer device 60. In practice, more or fewer components, or a combination of some components, or different components can be included. For example, the computer device 60 may further include an input/output device (such as a keyboard, a microphone, a camera, a loudspeaker, and a display screen, etc.).

Further, an embodiment of the present disclosure further provides a non-transitory computer-readable storage medium. The non-transitory computer-readable storage medium can be configured in the server in each of the above embodiments, and a computer program is stored in the non-transitory computer-readable storage medium. When the program is executed by a processor, the processing method for a radio frequency operation log according to the embodiments shown in FIG. 2 and FIG. 3 is implemented.

FIG. 7 is a schematic diagram of a processing system for a radio frequency operation log according to an embodiment of the present disclosure. As shown in FIG. 7, the system includes a server 701 and a plurality of radio frequency operation control devices 702 for respective different radio frequency operation systems.

The server 701 is configured to register the plurality of radio frequency operation control devices 702 as nodes in a blockchain network.

The radio frequency operation control device 702 is configured to implement the processing method for a radio frequency operation log according to the embodiments shown in FIGS. 2 and 3. The specific process for implementing the processing method for a radio frequency operation log by the radio frequency operation control device 702 can refer to relevant descriptions in embodiments shown in FIGS. 2 to 3, and will not be repeated here again.

Optionally, the radio frequency operation system, as shown in FIG. 1, includes a radio frequency operation control device, a syringe pump, a neutral electrode, a radio frequency ablation catheter and a physical characteristic data acquisition device. The radio frequency operation control device is a master control device, and the syringe pump, the neutral electrode, the radio frequency ablation catheter and the physical characteristic data acquisition device are electrically coupled to the radio frequency operation control device, and are slave devices to the radio frequency operation control device.

In the embodiment of the present disclosure, a running log and a radio frequency operation log are generated according to target running data and radio frequency operation data of a target device running in a radio frequency operation system acquired in real time; the running log is classified and stored in a running log file of the target device according to a preset first classification, and the operation log is classified and stored in an operation log file of the target device according to a preset second classification; and then the running log file and the operation log file are acquired and analyzed at a preset interval, to obtain maintenance data and operation result data of the radio frequency operation system. By the present embodiment, the generation, classified management, and analysis of logs of running data and radio frequency operation data of the radio frequency operation system are realized. Because the contents of the log include not only particular running data of the system, but also the radio frequency operation log of the system, the reference and pertinence of the log file is improved. Moreover, by means of classification and periodic analysis, the systematicity and efficiency in the management of the log file are also improved.

The above embodiments are described from different aspects, and aspects of some embodiments that are not described in detail can refer to relevant aspects of other embodiments.

Those skilled in the art would know that the exemplary modules / units and algorithm steps described in the embodiments disclosed herein can be implemented by electronic hardware, or a combination of computer software and electronic hardware. Depending on the specific application and design of the technical solutions, these functions can be realized by hardware or software. Those skilled in the art may use different methods to realize the described functions for each specific application, which should not be regarded as exceeding the scope of the present disclosure.

In the embodiments provided in the present disclosure, it should be understood that the disclosed devices / terminals and methods may be implemented in other manners. For example, the devices / terminals described above are only illustrative. For example, the division of the modules or units is only a division from the perspective of logical function, and in practice, other division manners can be used. For example, a plurality of modules or components can be combined or integrated into another system, or some features may be omitted or don't work. In another aspect, the mutual coupling or direct coupling or communication connection shown or discussed may be indirect coupling or communication connection between devices or units via some interfaces, in electrical, mechanical or other forms.

The units described as separate components may or may not be physically separated, and the components displayed as units may or may not be physical units, that is, they may be located in one place, or may be distributed on a plurality of network units. Part or all of the units can be used according to practical needs to achieve the purposes of the solutions of the embodiments.

In addition, the functional units in each embodiment of the present disclosure may be integrated into one processing unit, or may present separately physically, or two or more units may be integrated into one unit. The above-mentioned integrated units can be implemented in the form of hardware or in the form of software units.

If the integrated units are implemented in the form of software units and sold or used as independent products, they can be stored in a computer-readable storage medium. In view of this, all or part of the processes implemented in the methods of the above embodiments in the present disclosure may also be implemented by instructing related hardware through computer programs. The computer programs can be stored in a computer-readable storage medium, and when the computer program is executed by a processor, the steps of the above-mentioned various method embodiments can be implemented. The computer program includes computer program codes, and the computer program codes may be in the form of source code, object code, executable file or some intermediate form. Computer-readable media may include any entity or device, recording media, U disk, removable hard disk, magnetic disk, optical disk, computer memory, read-only memory (ROM), random access Memory (RAM), electrical carrier signal, telecommunication signal and software distribution medium, etc., that is capable of carrying computer program codes. It should be noted that the content contained in the computer readable media may be appropriately increased or decreased according to the requirements of legislation and patent practice in the jurisdiction. For example, in some jurisdictions, according to legislation and patent practice, the computer readable media does not include electrical carrier signal and telecommunication signal.

The above embodiments are only intended to explain the technical solutions of the present disclosure, rather than for limitation. Although the present disclosure has been described in detail with reference to the foregoing embodiments, those skilled in the art should understand that it is still possible to modify the technical solutions described in the foregoing embodiments, or make equivalent replacements for some or all of the technical features, and these modifications or replacements do not make the essence of the corresponding technical solutions deviate from the scope of the technical solutions of the various embodiments of the present disclosure, and should be included in the scope of the present invention.

## Claims

1. A processing method for a radio frequency operation log, applied in a computer device, comprising steps of:
acquiring, in real time, target running data and radio frequency operation data of a target device running in a radio frequency operation system;
generating a running log according to the target running data, and classifying and storing the running log in a running log file of the target device according to a preset first classification; and generating an operation log according to the radio frequency operation data, and classifying and storing the operation log in an operation log file of the target device according to a preset second classification; and
acquiring and analyzing the running log file at a preset interval, to obtain maintenance data of the radio frequency operation system; and acquiring and analyzing the operation log file at a preset interval, to obtain operation result data of the radio frequency operation system.

2. The method according to claim 1, wherein the first classification comprises an event log, an alert log, and an error log; and the step of classifying and storing the running log in a running log file of the target device according to a preset first classification comprises:
classifying and storing the running log in the running log file of the target device according to the event log, the alert log, and the error log, wherein
the event log comprises running data of an event with a preset target level triggered during normal running of the target device;
the alert log comprises running data when the target device is in a preset abnormal state; and
the error log comprises data when an error occurs in the target device.

3. The method according to claim 1, wherein the second classification comprises a start log, a suspension log, and an end log; and the step of classifying and storing the operation log in an operation log file of the target device according to a preset second classification comprises:
classifying and storing the operation log in the operation log file of the target device according to the start log, the suspension log, and the end log, wherein
the start log comprises operation parameter data of the target device when a radio frequency operation is started, and first physical characteristic data of a radio frequency operation object acquired by a temperature and/or impedance acquisition device;
the suspension log comprises operation parameter data of the target device when the radio frequency operation is suspended, and second physical characteristic data of the radio frequency operation object acquired by the temperature and/or impedance acquisition device; and
the end log comprises operation parameter data of the target device when the radio frequency operation is ended, and third physical characteristic data of the radio frequency operation object acquired by the temperature and/or impedance acquisition device.

4. The method according to claim 2 or 3, wherein the running log file and the operation log file comprise a timestamp field, a type field and a content field, wherein
the timestamp field is configured to store a timepoint corresponding to the log, the type field is configured to store classification information of the log, and the content field is configured to store the target running data or the radio frequency operation data.

5. The method according to claim 4, wherein the timepoint is a time difference from a preset timepoint.

6. The method according to claim 4, further comprising: saving the running log file and the operation log file in a file allocation table system.

7. The method according to claim 1, wherein the computer device is a device in the radio frequency operation system, and the computer device is a node in a blockchain network; and after the step of generating an operation log according to the radio frequency operation data, and classifying and storing the operation log in an operation log file of the target device according to a preset second classification, the method further comprises:
saving the running log file and the operation log file to the blockchain network to which the computer device is registered by invoking a preset smart contract for log storage; and
the step of acquiring and analyzing the running log file at a preset interval to obtain maintenance data of the radio frequency operation system and acquiring and analyzing the operation log file at a preset interval to obtain operation result data of the radio frequency operation system comprises:
acquiring and analyzing all the running log files and the operation log files from the blockchain network at the preset interval, to obtain the maintenance data and the operation result data.

8. The method according to claim 7, further comprising:
when a log file storage request sent from other nodes in the blockchain network is received, saving the running log file and the operation log file indicated by the log file storage request locally according to the smart contract for log storage; and
when a log file query request sent from other nodes in the blockchain network is received, returning response information comprising the running log file and the operation log file indicated by the log file query request according to the smart contract for log storage.

9. A processing apparatus for a radio frequency operation log, comprising:
a data acquisition module, configured to acquire, in real time, target running data and radio frequency operation data of a target device running in a radio frequency operation system;
a log classification module, configured to generate a running log according to the target running data, and classify and store the running log in a running log file of the target device according to a preset first classification; and generate an operation log according to the radio frequency operation data, and classify and store the operation log in an operation log file of the target device according to a preset second classification; and
a log analysis module, configured to acquire and analyze the running log file at a preset interval, to obtain maintenance data of the radio frequency operation system; and acquire and analyze the operation log file at a preset interval, to obtain operation result data of the radio frequency operation system.

10. A computer device, comprising:
a memory and a processor, wherein
the memory stores executable program codes; and
the processor is coupled to the memory, and configured to invoke the executable program codes stored in the memory, and implement the steps of the processing method for a radio frequency operation log according to any one of claims 1 to 8.

11. A processing system for a radio frequency operation log, comprising a server and a plurality of radio frequency operation control devices for respective radio frequency operation systems, wherein
the server is configured to register the plurality of radio frequency operation control devices as nodes in a blockchain network; and
the radio frequency operation control device is configured to implement the processing method for a radio frequency operation log according to any one of claims 1 to 8.

12. A non-transitory computer-readable storage medium, which stores a computer program, wherein when the computer program is executed by a processor, the processing method for a radio frequency operation log according to any one of claims 1 to 8 is implemented.
